# EUROPÄISCHE PATENTSCHRIFT

(11) **EP 1 868 977 B1**
(45) Veröffentlichungstag und Bekanntmachung des Hinweises auf die Patenterteilung: **14.10.2009**
(21) Anmeldenummer: 06725585.1
(22) Anmeldetag: 05.04.2006
(51) Int. Cl.: C07C 43/13, C07C 41/06, C08G 65/00, C08G 65/34, C11D 1/66

(54) **HERSTELLUNG VON (CO)TENSIDEN DURCH UMSETZUNG VON POLYOLEN MIT OLEFINEN**
PRODUCTION OF (CO)SURFACTANTS BY REACTING POLYOLS WITH OLEFINS
PREPARATION DE (CO)TENSIOACTIFS PAR MISE EN REACTION DE POLYOLS AVEC DES OLEFINES

(30) Priorität: 07.04.2005 DE 102005016152
(43) Veröffentlichungstag der Anmeldung: 26.12.2007
(73) Patentinhaber: BASF SE, 67056 Ludwigshafen (DE)
(72) Erfinder: STEPHAN, Jürgen, 68163 Mannheim (DE); RÖPER, Michael, 67157 Wachenheim (DE); HEIDEMANN, Thomas, 68519 Viernheim (DE); TRILLER, Michael, 68167 Mannheim (DE); TROPSCH, Jürgen, 67354 Römerberg (DE)
(74) Vertreter: Isenbruck, Günter
(86) Internationale Anmeldenummer: PCT/EP2006/061355
(87) Internationale Veröffentlichungsnummer: WO 2006/106124

(56) Entgegenhaltungen:
- EP-A- 0 649 829
- EP-A- 0 718 270
- EP-A- 1 160 232
- DE-A1- 4 021 511
- DE-A1- 4 118 568
- DE-A1- 4 222 183
- DE-A1- 10 036 423
- US-A- 1 968 033
- US-A- 2 235 785
- US-A- 3 170 915
- US-A- 4 404 408
- US-A- 4 694 084
- US-A- 5 153 179
- US-A1- 2004 186 325

## Beschreibung

Die vorliegende Erfindung betrifft ein Verfahren zur Herstellung von Polyolalkylethern, die so erhaltenen Polyolalkylether und die Verwendung dieser Polyolalkylether als Tenside z.B. in Wasch- und Reinigungsmitteln.

Bislang wurden nichtionische Tenside zumeist durch Addition von Alkylenoxiden an Tensidalkohole oder Fettalkohole hergestellt. Die dazu benötigten Tensidalkohole können erhalten werden durch
- Hydroformylierung und anschließende Hydrierung von Olefinen mit Kohlenmonoxid und Wasserstoff. Nachteil dieses Verfahrens sind der hohe Kostenaufwand für Reaktoren und Sicherheitsmaßnahmen, sowie die Erzeugung von Synthesegas in einem vorgelagerten, zusätzlichen Verfahrensschritt.
- Direkte Oxidation von Paraffinen in Gegenwart von Borsäure (Bashkirov-Oxidation). Bei diesem Verfahren entstehen sekundäre Alkohole, die schwer zu alkoxylieren sind. Die Verfügbarkeit linearer Paraffine ist nicht immer gewährleistet.
- Zieglerverfahren: In der so genannten Alfol-Synthese werden die aus Ethylen und Triethylaluminium hergestellten Trialkylaluminium-Verbindungen mit Luft zu den entsprechenden Alkoxiden oxidiert und anschließend zu Alkoholen und Al₂O₃ hydrolysiert. Problematisch sind bei diesem Verfahren der Umgang mit Aluminiumalkylverbindungen und die gekoppelte Produktion von Aluminiumoxid.

Alternativ können Tensidalkohole durch die folgenden Verfahren hergestellt werden:
- Hydratisierung von Olefinen. Die Herstellung von Tensidalkoholen durch die Hydratisierung von Olefinen ist aufgrund der ungünstigen Gleichgewichtslage und der niedrigen Reaktionsgeschwindigkeit nur schlecht möglich.

Zur Herstellung von Tensiden ist die Addition von primären Alkoholen an Doppelbindungen nur schlecht möglich, da nicht aktivierte Alkohole wegen ihrer mangelnden Nukleophilie nur langsam reagieren. Die Aktivierung der primären Alkohole in der Merkurierung/Oximerkurierung beinhaltet den Nachteil des Einsatzes von toxischen Quecksilber-Verbindungen.

Die Umsetzung von (Polyalkylen)-diolen mit Olefinen, bevorzugt Umsetzung von Monoethylenglykol mit Olefinen, ist bekannt.

So offenbart EP 0 747 339 B2 ein Verfahren zur Herstellung von Polyalkylenglykolmonoalkylethem aus Olefinen und (Poly)alkylenglykolen. Dabei werden Olefine wie Octen, Decen, Dodecen, Tetradecen, Hexadecen, Octadecen mit Alkoholen ausgewählt aus Monoethylenglykol, Diethylenglykol, Triethylenglykol, Monopropylenglykol, 1,3-Propandiol und anderen in Gegenwart von kristallinen Metallosilikaten als Katalysatoren umgesetzt.

EP 0 846 671 A2 offenbart ein Verfahren zur Herstellung von (Poly)alkylenglykolmonoalkylethern durch Umsetzung von Olefinen ausgewählt aus C₈- bis C₃₀-Olefinen, die die Doppelbindung in α-Position oder in einer internen Position aufweisen, mit Alkoholen wie Monoethylenglykol, Diethylenglykol, Monopropylenglykol, 1,3-Propandiol und anderen in Gegenwart von Harzen, Zeolithen oder homogenen sauren Katalysatoren.

EP 0 850 907 B1 offenbart ein Verfahren zur Herstellung von höheren sekundären Alkoholalkoxylat-Verbindungen durch Umsetzung von langkettigen Olefinen mit 8 bis 30 Kohlenstoffatomen, wobei die Doppelbindung in α-Position oder intern vorlieben kann, mit 1 bis 50 Äquivalenten eines C₂- bis C₈-Alkylenoxids.

Ein Nachteil an den genannten Verfahren des Standes der Technik ist, dass die als Substrate eingesetzten Glykole in einem weiteren Verfahrensschritt hergestellt werden müssen. Des Weiteren sind die dort als Nebenprodukt gewonnenen Zweifach-Additionsprodukte (Dialkylether) wertlos, da ihre Hydrophilie für den Einsatz als Tenside unzureichend ist.

DE 195 44 413 A1 offenbart ein Verfahren zur Herstellung von Polyolalkylethern durch Reaktion von Verbindungen, die wenigstens zwei Hydroxyfunktionen aufweisen, in Gegenwart saurer Katalysatoren bei Temperaturen von 50 bis 120 °C und Drücken von 50 bis 30 bar mit Olefinen, wobei die Reaktion in der Flüssigphase in Gegenwart eines Lösungsmittels durchgeführt wird. Es werden kurzkettige Olefine wie iso-Buten eingesetzt.

DE 44 45 635 A1 offenbart ein Verfahren zur Herstellung von Polyolalkylethern, bei dem man Polyhydroxyverbindungen, ausgewählt aus der Gruppe bestehend aus Alkylenglykolen, Glycerin, Trimethylolpropan und Pentaerythrit in Gegenwart von sauren Katalysatoren bei Temperaturen von 50 bis 120 °C und Drücken von 50 bis 30 bar mit kurzkettigen Olefinen wie C₃- bis C₆-α-Olefinen oder C₄-C₁₀-Vinyliden-Olefinen, umsetzt. Zur Erzielung eines hohen Umsatzes in kurzer Reaktionszeit wird die Reaktion in der Flüssigphase in Gegenwart eines Lösungsmittels durchgeführt.

DE 42 22 183 A1 offenbart ein Verfahren zur Herstellung von Polyolalkylethern durch Umsetzung von Polyhydroxyverbindungen, ausgewählt aus der Gruppe bestehend aus Alkylenglykolen, Glycerin, Oligoglycerinen, Trimethylolpropan, Pentaerythrit, 1,12-Dodecandiol und Sorbitol in Gegenwart von sauren Katalysatoren mit kurzkettigen C₃-bis C₈-α-Olefinen und C₄- bis C₁₄-Vinylidenolefinen. Als Katalysatoren werden heterogene Katalysatoren wie saure Ionentauscher oder Zeolithe, sowie homogene saure Katalysatoren eingesetzt. DE 42 22 183 A1 offenbart weiterhin die Verwendung der genannten Polyolalkylether als Lösungsmittel in Reinigungsmitteln, Farben, Lacken und als Treibstoffadditive.

EP 0 649 829 A1 offenbart ein Verfahren zur Herstellung von Glycerolethem, in dem Glycerol mit geeigneten Olefinen in Gegenwart eines Zeolith-Katalysators umgesetzt wird. Geeignete Olefine weisen 4 bis 11 Kohlenstoffatome auf.

Die genannten Verfahren offenbaren die Umsetzung von Polyhydroxyverbindungen mit kurzkettigen Olefinen. Aufgrund des schwach ausgebildeten hydrophoben Charakters der Seitenketten sind die offenbarten Verbindungen, die aus der Umsetzung von kurzkettigen Olefinen mit Polyhydroxyverbindungen herrühren, nur unzulänglich als Tenside geeignet.

Aufgabe der vorliegenden Erfindung ist es, ein Verfahren bereitzustellen, durch das effizient und in einem Schritt Verbindungen hergestellt werden können, die besonders gut als Tenside in Reinigungsmitteln geeignet sind.

Diese Aufgabe wird gelöst durch ein Verfahren zur Herstellung von Polyolalkylethern durch Umsetzung von wenigstens drei Hydroxyfunktionalitäten enthaltenden Verbindungen mit Olefinen in Gegenwart von sauren Katalysatoren bei Temperaturen von 20 bis 250 °C und Drücken von 0,5 bis 10 bar, wobei die Olefine der allgemeinen Formel (I) entsprechen 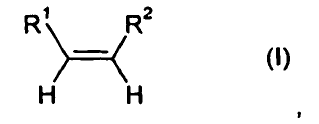 worin R¹ Wasserstoff und R² linearer oder verzweigter Kohlenstoffrest mit 7 bis 28 Kohlenstoffatomen bedeuten,
oder
R¹ und R² jeweils linearer oder verzweigter Kohlenstoffrest mit 1 bis 27 Kohlenstoffatomen, wobei die Summe der Kohlenstoffanzahl von R¹ und R² höchstens 28 beträgt, bedeuten, wobei die wenigstens drei Hydroxyfunktionalitäten enthaltende Verbindung ausgewählt ist aus Sorbitol, Trimethylolpropan, Pentaerythrit, Verbindungen der Formel (IV), (V), (VI), (IX) und Mischungen davon 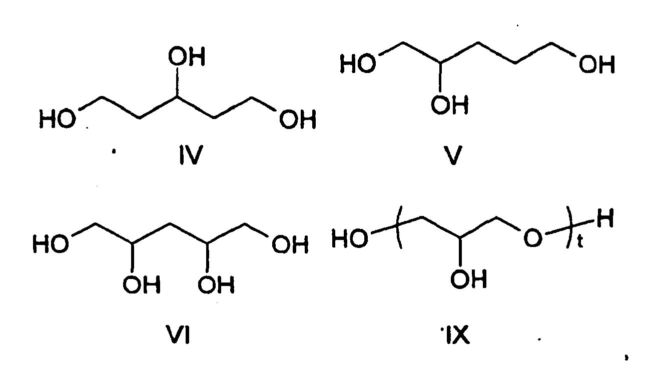 wobei t 1 bis 20 ist und die Verbindungen der Formel (IX) linear oder verzweigt sein können.

Die Etherbindungen in den Verbindungen der allgemeinen Formel (IX) können, wie gezeigt, über die primären Hydroxyfunktionalitäten erfolgen, so dass lineare Verbindungen der allgemeinen Formel (IX) erhalten werden. Wird die Etherbindung in den Verbindungen der allgemeinen Formel (IX) über mindestens eine sekundäre Hydroxyfunktionalität gebildet, liegen verzweigte Verbindungen vor.

Die wenigstens drei Hydroxyfunktionen enthaltende Verbindung ist ausgewählt aus der Gruppe bestehend aus Glycerin, Sorbitol, Trimethylolpropan, Pentaerythrit, Verbindungen der Formel (IV), (V), (VI), (IX) und Mischungen davon.

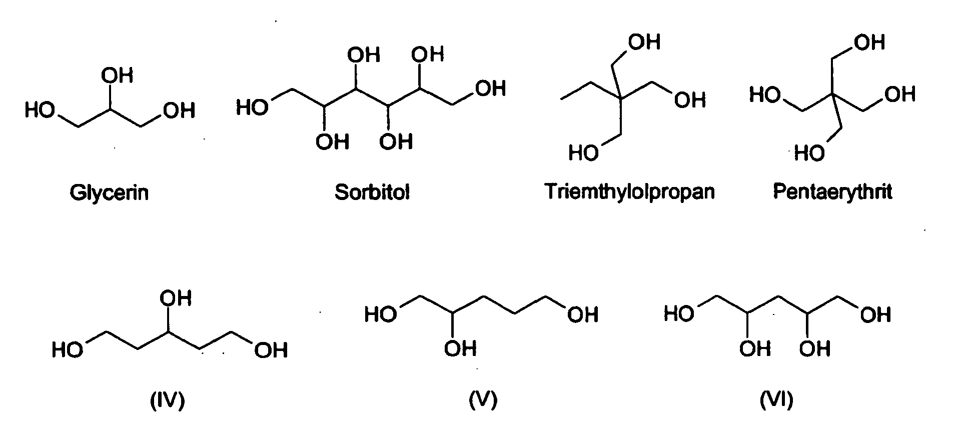

Ganz besonders bevorzugt ist die Verbindung ausgewählt aus der Gruppe bestehend aus Glycerin, Sorbitol, Trimethylolpropan, Pentaerythrit, Verbindungen der allgemeinen Formel (IX) und Mischungen davon.

Die wenigstens drei Hydroxyfunktionen enthaltenden Verbindungen können nach dem Fachmann bekannten Verfahren hergestellt oder erhalten werden.

Natürliches Glycerin fällt in großem Maßstab als Nebenprodukt bei der Hydrolyse, Verseifung oder Umesterung von Fetten an. In diesem Zusammenhang ist vor allem die weltweit ständig zunehmende Herstellung von "Biodiesel" zu nennen, bei dem Triglyceride (Fette, Öle) mit Methanol zu den entsprechenden Fettsäuremethylestern und dem als Kopplungsprodukt anfallenden Glycerin umgesetzt werden.

Synthetisches Glycerin kann über die folgenden Routen hergestellt werden, die von Propen ausgehen:

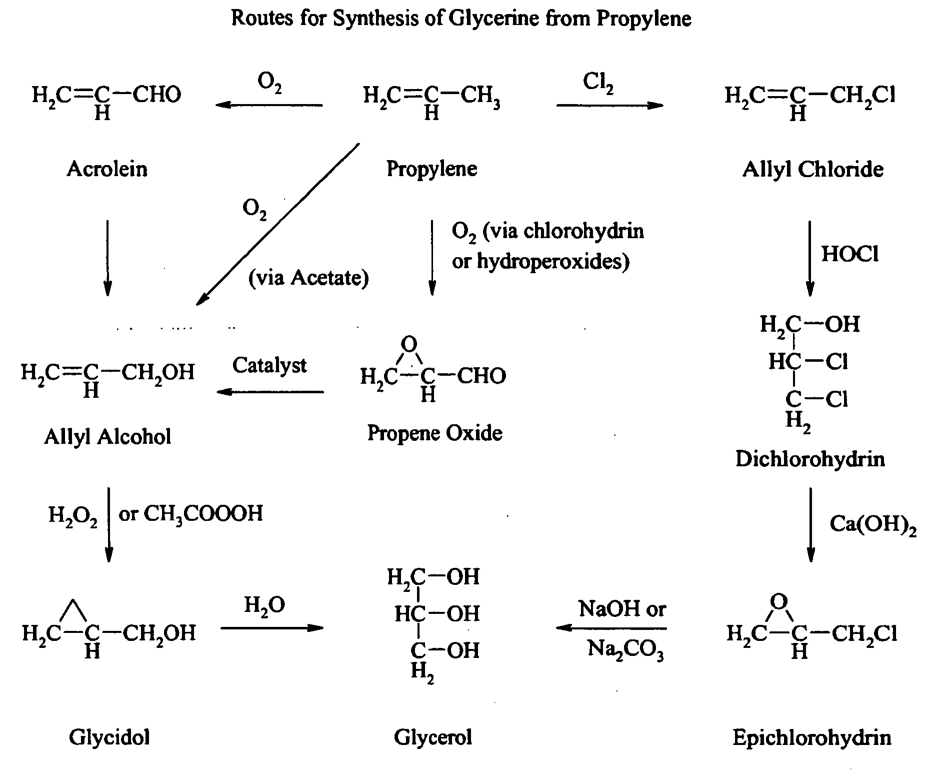

Sorbitol wird erhalten durch nickel- oder rhodium-katalysierte Hydrierung von Glucose.

Pentaerythrit wird hergestellt durch Kondensation von Formaldehyd und Acetaldehyd in Gegenwart einer Base. Die entstehende Pentaerythrose reagiert per Cannizaroreaktion zu Pentaerythrit und Natriumformiat.

Trimethylolpropan wird hergestellt durch Kondensation von Formaldehyd und Butyraldehyd in Gegenwart einer Base, der entstehende 2,2-Dimethylolbutyraldehyd wird in einer Cannizaroreaktion mit Formaldehyd in Gegenwart von NaOH zu Trimethylolpropan umgesetzt.

Diglycerin, t = 2 in Formel (IX), kann hergestellt werden durch Erhitzen von Gylcerin auf 290 - 295 °C wie in DE 181754 beschrieben, durch Erhitzen von Glycerin mit Erdalkalioxiden oder Wasserglas, wie in DE 494430 und DE 494431 beschrieben oder durch alkalische Hydrolyse von Epichlorhydrin.

Polyglycerine der allgemeinen Formel (IX) können erhalten werden durch Erhitzen von Glycerin mit Alkali nach DE 198768 oder US 3,637,774, durch Reaktion von Isopropylidenglycerin mit α-Monochlorhydrin (basisch) und anschließender Hydrolyse nach US 5,243,086 oder DE 4132171, durch Erhitzen von Glycerin mit Glycerincarbonat basisch oder sauer katalysiert nach JP 10072392 oder heterogen katalysiert nach JP 10072393, durch Erhitzen von Glycerin mit Epichlorhydrin basisch katalysiert nach US 4,960,953, oder durch Reaktion von Glycidol mit Glycerin nach A. Kleemann, R. Wagner, Hüthig-Verlag, Heidelberg, 1981, 62-63.

Die in dem erfindungsgemäßen Verfahren eingesetzten Olefine entsprechen der allgemeinen Formel (I) 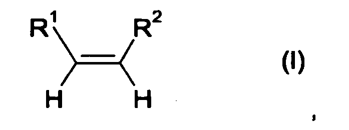 worin R¹ Wasserstoff und R² linearer oder verzweigter Kohlenstoffrest mit 7 bis 28 Kohlenstoffatomen,
oder
R¹ und R² jeweils linearer oder verzweigter Kohlenstoffrest mit 1 bis 27 Kohlenstoffatomen, wobei die Summe der Kohlenstoffatome von R¹ und R² höchstens 28 beträgt, bedeuten.

Die Olefine, die durch die allgemeine Formel (I) beschrieben werden, sind α-Olefine mit 9 bis 30 Kohlenstoffatomen, oder Olefine mit 4 bis 30 Kohlenstoffatomen, die die Doppelbindung in interner Position aufweisen.

Geeignete Verfahren zur Herstellung der erfindungsgemäß einsetzbaren Olefine sind dem Fachmann bekannt.

α-Olefine mit einer Kettenlänge von wenigstens 9 Kohlenstoffatomen werden nahezu ausschließlich durch Separierung des α-Olefins mit der gewünschten Kettenlänge aus Gemischen, die durch Oligomerisierung von Ethen hergestellt werden (so genannter Full-range-Prozess im Gegensatz zu on-purpose-Prozessen). Dazu sind beispielsweise die Verfahren der Chevron Philipps (Verwendung eines AlEt₃-Katalysators in verdünnter Lösung), der BP (konzentrierte AlEt₃-Lösung), der Idemitsu (Verwendung eines Al/Zr-Komplexes) oder der Shell (Ligand-modifiziertes Nickelsystem) bekannt. Im Shell-Prozess erfolgt darüber hinaus noch eine Sequenz aus Isomerisierung und nachfolgender Metathesereaktion der Olefine, um so interne Olefine zu erhalten. Die derart erhaltenen internen Olefine sind ebenfalls zum erfindungsgemäßen Einsatz geeignet.

Neue Full-range-Prozesse sind beispielsweise das AlphaSelect^{®}-Verfahren der Axens oder das Alpha-Sablin^{®}-Verfahren von Linde/Sabic, die Katalysatoren basierend auf Zirkonium/Aluminium einsetzen. Weiterhin ist das Verfahren der UOP/Dow zu nennen (Linear-1^{®}).

On purpose Prozesse für α-Olefine mit mehr als 8 Kohlenstoffatomen sind bislang nicht technisch realisiert. Es existieren Anmeldungen der BASF-Aktiengesellschaft für ein Verfahren zur Herstellung von 1-Decen (EP 10103309, EP 10128048).

Des Weiteren können eingesetzt werden: (lineare) interne Olefine aus dem SHOP-Verfahren oder auch verzweigte Olefine. Diese gewinnt man beispielsweise durch Trimerisierung von n-Butenen (EP 1030825) oder auch Dimerisierung von Hexenen, wie beschrieben in EP 1268370 (BASF Aktiengesellschaft), WO 00/69795 (BASF Aktiengesellschaft) oder EP 1159236.

In einer bevorzugten Ausführungsform werden Olefine ausgewählt aus der Gruppe bestehend aus internen, verzweigten Olefinen mit der Kettenlänge C₁₂, linearen oder verzweigten, bevorzugt linearen, α-Olefinen der Kettenlänge C₁₂ und C₁₄, 1-Dodecen und/oder 1-Tetradecen, und Mischungen davon in das erfindungsgemäße Verfahren eingesetzt.

In einer besonders bevorzugten Ausführungsform werden ein C₁₂-, ein C₁₄-α-Olefin oder eine Mischung eines C₁₂- und eines C₁₄-α-Olefins eingesetzt. Eine bevorzugte C₁₂-, C₁₄-α-Olefin-Mischung enthält 95 Gew.-% α-Olefine, davon 67±3 Gew.-% 1-Dodecen and 33±3 Gew.-% 1-Tetradecen.

Interne, verzweigte Olefine der Kettenlänge C₁₂ können hergestellt werden durch Dimerisierung von Hexenen wie beschrieben in EP 1268370 (BASF Aktiengesellschaft), WO 00/69795 (BASF Aktiengesellschaft), EP 1159236 (BASF Aktiengesellschaft), oder durch Trimerisierung von Butenen wie in EP 1030825 beschrieben.

In einer weiteren bevorzugten Ausführungsform wird ein Internes, leicht verzweigtes Olefingemisch mit folgender Zusammensetzung eingesetzt:
10 - 18 Gew.-% von n-Dodecan abgeleitetes Olefin,
25 - 40 Gew.-% von 5-Methyl-n-undecan abgeleitetes Olefin,
25 - 40 Gew.-% von 4-Ethyl-n-decan abgeleitetes Olefin,
2 - 8 Gew.-% von 5,6-dimethyl-n-decan abgeleitetes Olefin,
5 - 12 Gew.-% von 5-Ethyl-6-methyl-n-nonan abgeleitetes Olefin,
1 - 5 Gew.-% von 4,5-Di-ethyl-n-octan abgeleitetes Olefin
und höchstens 5 Gew.-% anderer Kohlenwasserstoffe.

Das erfindungsgemäße Verfahren wird in Gegenwart von sauren Katalysatoren durchgeführt. Die sauren Katalysatoren können in der Reaktionsmischung heterogen oder homogen vorliegen, wobei Heterogen-Katalysatoren bevorzugt sind.

Geeignete homogene Katalysatoren sind starke Säuren, beispielsweise Alkylschwefelsäuren, para-Toluolsulfonsäure, allgemein Alkyl- und Arylsulfonsäuren, Phosphorsäure, Trifluormethansulfonsäure, HF, SO₃, Borsäure, Perchlorsäure oder Lewis-Säuren, beispielsweise BF₃, BCl₃, AlBr₃, FeCl₃, SnCl₄, SbCl₅, AsF₅, AsF₃, TiCl₄, AlMe₃ oder verwandte Verbindungen.

Geeignete heterogene Katalysatoren für das vorliegende Verfahren sind starke Säuren wie Ionentauscher auf Divinylbenzolbasis mit Sulfonsäuregruppen (z.B. Amberlyst 15 der Firma Rohm & Haas), Heteropolysäuren und deren Salze (z.B. H₄SiMo₁₂O₄₀, H₃PMo₁₂O₄₀, H₃PW₁₂O₄₀) auf Trägermaterialien wie SiO₂, Al₂O₃, TiO₂, ZrO₂ usw. sowie saure Mischoxide (z.B. WO₃/TiO₂).

Des Weiteren geeignet sind poröse Metallo-, insbesondere Alumosilikate. Dies umfasst mesoporöse Alumosilikate wie MCM-41, SBA-15, MSU-S, MAS-5, MAS-7, MAS-9. Diese Materialien sind in "Catalysis Surveys from Asia, 2004, Vol. 8., No. 3, S.151 ff. und den darin enthaltenen Referenzen beschrieben. Besonders geeignet sind kristalline Metallosilikate wie Alumosilikate, Ferrosilikate, Borosilikate oder Gallosilikate.

In einer bevorzugten Ausführungsform wird als saurer Katalysator ein Zeolith eingesetzt.

Beispiele für Zeolithe, die erfindungsgemäß eingesetzt werden können, sind Zeolithe der Strukturtypen FER (z.B. Ferrierit), MFI und MEL (Pentasile, z.B. ZSM-5, ZSM-11), FAU (z.B. X, Y, USY), LTL (Linde Typ L), MOR (Mordenit), BEA (β-Zeolith), MTW (z.B. ZSM-12), GME (z.B. Gmelinit), MAZ (Mazzit) und MWW (z.B. MCM-22). Aufgrund ihrer vorteilhaften katalytischen Eigenschaften sind aus dieser Gruppe BEA-, MFI-, MEL-MOR-, FAU- und MWW-Zeolithe bevorzugt. Ganz besonders bevorzugt sind BEA-Zeolithe.

Diese Zeolithe werden bevorzugt in der H-Form eingesetzt, können jedoch in Nicht-Gitter-Positionen auch Metallionen aus den Gruppen IA und IIA des Periodensystem der Elemente (CAS-Nomenklatur) sowie Ti, Cr, Mn, Fe, Ni, Cu, Co, Ag, Zn oder La enthalten. Die Metallionen können während der Zeolithsynthese eingebaut oder danach durch Metallionenaustausch oder durch Imprägnierung aufgebracht werden.

Der Katalysator kann als feines Pulver in Suspension direkt verwendet werden, bei Zeolithen sind dies in der Regel Teilchengrößen zwischen 100 nm und einigen µm.

Ebenso können diese Katalysatoren jedoch zusammen mit Bindermaterialien zu Formkörpern verformt werden. Als Binder eignen sich besonders Tone, Aluminiumoxide wie z.B. Pural und Siliziumoxide wie z.B. Silres. Als Formkörper eignen sich Tabletten, Stränglinge, Ringe, Rippstränge, Stern- oder Wagenradextrudate. Gebräuchlich sind Durchmesser von 0,1 - 5 mm.

Die Katalysatoren besitzen spezifische Oberflächen von 30 bis 2000 m²/g, bevorzugt 100 bis 900 m²/g. Das Volumen der Poren mit Durchmesser 2-20 nm beträgt typischerweise 0,05 - 0,5 ml/g, bevorzugt 0,1 - 0,3 ml/g, das der Poren von 20 - 200 nm typischerweise 0,005 bis 0,2 ml/g, bevorzugt 0,01 bis 0,1 ml/g, das der Poren von 200 - 2000 nm typischerweise 0,05 - 0,5 ml/g, bevorzugt 0,05 bis 0,3 ml/g.

Deaktivierte Katalysatoren können zum Beispiel durch Abbrennen in Luft oder Magerluft bei 250 - 550°C reaktiviert werden. Alternativ zum Abbrennen ist eine Behandlung mit bei tieferer Temperatur sowohl in der Flüssig- als auch in der Gasphase mit Mineralsäuren wie z.B. Schwefelsäure oder Salzsäure, mit organischen Lösungsmitteln oder mit oxidierend wirkenden Verbindungen möglich, hier sind insbesondere NOₓ, H₂O₂ und die Halogene zu nennen. Die Regenerierung kann direkt im Reaktor oder extern erfolgen.

Die erfindungsgemäß einsetzbaren Katalysatoren sind kommerziell erhältlich oder nach dem Fachmann bekannten Methoden herstellbar.

Das Verfahren wird bei einer Temperatur von 20 bis 250 °C, bevorzugt 40 bis 200 °C, besonders bevorzugt 80 bis 180 °C durchgeführt.

Das erfindungsgemäße Verfahren wird bei einem Druck von 0,5 bis 10 bar, bevorzugt 0,6 bis 5 bar, besonders bevorzugt bei Atmosphärendruck durchgeführt.

Das erfindungsgemäße Verfahren kann in Anwesenheit oder in Abwesenheit eines Lösungsmittels durchgeführt werden. In einer bevorzugten Ausführungsform wird das erfindungsgemäße Verfahren in Anwesenheit eines Lösungsmittels durchgeführt.

Als geeignete Lösungsmittel sind einwertige Alkohole mit 1 bis 6 Kohlenstoffatomen, niedermolekulare Ether, aromatische Verbindungen wie Benzol oder Toluol, chlorierte Lösungsmittel wie Methylenchlorid, Chloroform oder Tetrachlorkohlenstoff, 1,4-Dioxan, 1,3-Dioxan, Sulfolan, THF oder Verbindungen der allgemeinen Formel (VII) 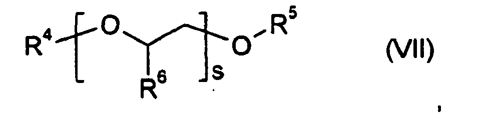 worin R⁴, R⁵ und R⁶ unabhängig von einander Wasserstoff, C₁-C₁₀-Alkyl, -Alkenyl oder -Alkinyl bedeuten, bevorzugt bedeuten R⁴ und R⁵ C₁-C₃-Alkyl, weiterhin bevorzugt bedeutet R⁶ Wasserstoff oder Methyl, besonders bevorzugt Wasserstoff, s bedeutet eine ganze Zahl von 1 bis 5, bevorzugt 2 bis 4, genannt. Es können auch Mischungen der genannten Lösungsmittel eingesetzt werden. Für die Umsetzung von festen Polyolkomponenten, wie Sorbitol, kann auch z.B. Glycerin als Lösungsmittel verwendet werden, was zu Produktmischungen führt. Des Weiteren sind für Sorbit polare Lösungsmittel geeignet, z.B. DMSO oder Acetonitril.

Bevorzugt werden 1,4-Dioxan, 1,3-Dioxan, Diglyme, Triglyme oder Mischungen davon als Lösungsmittel eingesetzt. Es können auch Mischungen der genannten Lösungsmittel eingesetzt werden.

Das erfindungsgemäße Verfahren wird in einer bevorzugten Ausführungsform in inerter Atmosphäre durchgeführt. Als inerte Gase können Stickstoff und/oder Edelgase, bevorzugt Helium und/oder Argon, eingesetzt werden. Bevorzugt wird eine inerte Atmosphäre bestehend aus Stickstoff eingesetzt.

Als Reaktionsgefäße sind für das erfindungemäße Verfahren alle dem Fachmann bekannten Gefäße einsetzbar, beispielsweise kontinuierlich, halb- oder diskontinuierlich betriebene Glas- oder Metall-Rührkessel bzw. Druckautoklaven.

Das erfindungsgemäße Verfahren kann sowohl kontinuierlich als auch diskontinuierlich durchgeführt werden. Bei kontinuierlicher Fahrweise werden die Substrate kontinuierlich dem Reaktor zugeführt, und die Produkte werden kontinuierlich aus dem Reaktor abgeführt, so dass die Konzentrationen der einzelnen Komponenten im Reaktor konstant bleiben.

Das Reaktionsprodukt kann nach dem Fachmann bekannten Verfahren aufgearbeitet und/oder gereinigt werden. Zur Aufarbeitung kann die das Produkt enthaltende Phase durch Abdekantieren oder mittels eines Scheidetrichters abgetrennt werden. Des Weiteren kann die Abtrennung des Produkts auch destillativ erfolgen. Auch die Reinigung des Produkts kann destillativ erfolgen. Nicht umgesetzte Substrate können nach ihrer Abtrennung erneut in die Reaktion eingesetzt werden.

Die vorliegende Erfindung betrifft des Weiteren Polyolalkylether, abgeleitet von Verbindungen mit wenigstens drei Hydroxyfunktionalitäten, ausgewählt aus Verbindungen der Formel (IV), (V), (VI), (IX) und Mischungen davon 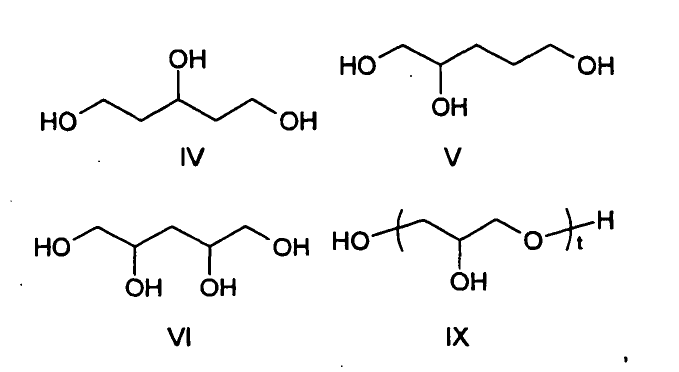 wobei t 2 bis 20 ist, und die Verbindungen der Formel (IX) linear oder verzweigt sein können, wobei höchstens alle bis auf eine Hydroxyfunktionalität durch eine Gruppierung der allgemeinen Formel (VIII) 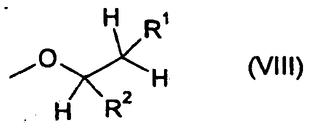 ersetzt sind, und die Reste R¹ und R² die gleichen Bedeutungen wie in den Verbindungen der allgemeinen Formel (I) aufweisen.

Die Gruppierung der allgemeinen Formel (VIII) leitet sich von den Olefinen der allgemeinen Formel (I) ab.

In einer bevorzugten Ausführungsform leitet sich die Gruppierung der allgemeinen Formel (VIII) von Olefinen ab, ausgewählt aus der Gruppe bestehend aus internen, verzweigten Olefinen mit der Kettenlänge C₁₂, linearen oder verzweigten, bevorzugt linearen, α-Olefinen der Kettenlänge C₁₂ und C₁₄, 1-Dodecen und/oder 1-Tetradecen, und Mischungen davon.

In einer besonders bevorzugten Ausführungsform leitet sich die Gruppierung der allgemeinen Formel von C₁₂-, C₁₄-α-Olefinen oder von einer Mischung eines C₁₂- und eines C₁₄-α-Olefins ab.

Die vorliegende Erfindung betrifft auch Wasch- und Reinigungsmittel, die die erfindungsgemäßen Polyolalkylether enthalten.

Diese Wasch- und Reinigungsmittel können in Pulver-, Granulat-, Extrudat- oder Tablettenform vorlieben.

In Waschmitteln können die erfindungsgemäßen Polyolalkylether mit den üblichen, einem Fachmann bekannten Zusatzstoffen in Mengen von 0,1 bis 40 Gew.-%, bevorzugt 0,5 bis 30 Gew.-%, besonders bevorzugt 1 bis 20 Gew.-%, kombiniert werden. Beispiele für geeignete Zusatzstoffe umfassen:
- Builder und Cobuilder, beispielsweise Polyphosphate, Zeolithe, Polycarboxylate, Phosphonate, Citrate, Komplexbildner,
- Ionische Tenside, beispielsweise Alkylbenzolsulfonate, α-Olefinsulfonate und andere Alkoholsulfate/-ethersulfate,
- andere nichtionische Tenside, beispielsweise Alkylaminoalkoxylate und Alkylpolyglykoside, amphotere Tenside, beispielsweise Alkylaminoxide, Betaine,
- optische Aufheller,
- Farbübertragungsinhibitoren, beispielsweise Polyvinylpyrrolidon,
- Stellmittel, beispielsweise Natriumsulfat, Magnesiumsulfat,
- Soil Release Agenzien, beispielsweise Polyether/-ester, Carboxymethylcellulose,
- Inkrustierungsinhibitoren, beispielsweise Polyacrylate, Copolymere aus Acrylsäure und Maleinsäure,
- Bleichsysteme, bestehend aus Bleichmitteln, beispielsweise Perborat oder Percarbonat, plus Bleichaktivatoren, beispielsweise Tetraacetylethylendiamin, plus Bleichstabilisatoren,
- Parfüm,
- Schaumdämpfer, beispielsweise Silikonöle, Alkoholpropoxilate (vor allem in Flüssigwaschmitteln),
- Enzyme, beispielsweise Amylasen, Lipasen, Proteasen oder Carbonylasen,
- Alkalispender, beispielsweise Pentanatriummetasilicat oder Natriumcarbonat.

Weitere, dem Fachmann bekannte Bestandteile können ebenfalls enthalten sein.

Die pulverförmigen Waschmittel haben in der herkömmlichen Form ein durchschnittliches Schüttgewicht von ca. 450 g/l. Kompakt- oder Ultra-Kompaktwaschmittel weisen ein Schüttgewicht von > 600 g/l auf.

Flüssige Waschmittel können zusätzlich Lösungsmittel enthalten, beispielsweise Ethanol, Isopropanol, 1,2-Propylenglykol oder Butylenglykol.

Gelförmige Waschmittel enthalten zusätzlich Verdicker, beispielsweise Polysaccharide und schwach vernetzte Polycarboxylate, beispielsweise die Carbopol^{®}-Marken der BF Goodrich.

Bei tablettenförmigen Waschmitteln werden weitere Zusatzstoffe benötigt. Dies sind beispielsweise Tablettierhilfsmittel, beispielsweise Polyethylenglykole mit Molmassen > 1000 g/mol oder Polymerdispersionen. Benötigt werden auch Tablettensprengmittel, beispielsweise Cellulosederivate, vernetztes Polyvinylpyrrolidon, vernetzte Polyacrylate oder Kombinationen aus Säuren, beispielsweise Zitronensäure, mit Natriumcarbonat.

In Reinigern für harte Oberflächen, beispielsweise sauren Reinigern, alkalischen Reinigern, Neutralreinigern, Maschinengeschirrreinigung, zur Metallentfettung, Glasreiniger, Fußbodenreiniger, werden die erfindungsgemäßen Polyolalkylether kombiniert mit den nachfolgend aufgeführten, in Mengen von 0,01 bis 40 Gew.-%, bevorzugt 0,1 bis 20 Gew.-%, vorhandenen Zusatzstoffen:
- ionische Tenside, beispielsweise Alkylbenzolsulfonate, a-Olefinsulfonate, andere Alkoholsulfonate/-ethersulfonate, Sulfosuccinate,
- andere nichtionische Tenside, beispielsweise Alkylaminalkoxilate und Alkylpolyglucoside,
- amphotere Tenside, beispielsweise Alkylaminoxide, Betaine,
- Builder, beispielsweise Polyphosphonate, Polycarboxylate, Phosphonate, Komplexbildner,
- Dispergiermittel, beispielsweise Naphthalinsulfonsäurekondensate, Polycarboxylate
- pH-regulierende Verbindungen, beispielsweise Alkalien wie NaOH, KOH oder Pentanatriummetasilikat oder Säuren, etwa Salzsäure, Phosphorsäure, Amidoschwefelsäure, Zitronensäure,
- Enzyme, beispielsweise Lipasen, Amylasen, Proteasen, Carboxylasen,
- Parfüm,
- Farbstoffe,
- Biozide, beispielsweise Isothiazolinone, 2-Bromo-2-nitro-1,3-propan-diol,
- Bleichsysteme, bestehend aus Bleichmitteln, beispielsweise Perborat, Percarbonat, plus Bleichaktivatoren, beispielsweise Tetraacetylethylendiamin, plus Bleichaktivatoren,
- Solubilisatoren, beispielsweise Cumolsulfonate, Toluolsulfonate, kurzkettige Fettsäuren, Phosphorsäurealkyl/-arylester,
- Lösungsmittel, beispielsweise kurzkettige Alkyloligoglykole, Alkohole, beispielsweise Ethanol oder Propanol, aromatische Lösungsmittel, beispielsweise Toluol oder Xylol, N-Alkylpyrrolidone, Alkylencarbonate,
- Verdicker, beispielsweise Polysaccharide und schwach vernetzte Polycarboxylate, beispielsweise Carbopol^{®}-Marken der BF Goodrich.

Diese Reiniger für harte Oberflächen sind gewöhnlich, aber nicht ausschließlich wässrig und liegen in der Form von Mikroemulsionen, Emulsionen oder in Lösung vor.

Sollten sie in fester Form vorliegen, können zusätzlich Stellmittel wie oben beschrieben eingesetzt werden.

Bei tablettenförmigen Reinigern werden weitere Zusatzstoffe benötigt. Dies sind beispielsweise Tablettierhilfsmittel, beispielsweise Polyethylenglykole mit Molmassen > 1000 g/mol oder Polymerdispersionen. Benötigt werden auch Tablettensprengmittel, beispielsweise Cellulosederivate, vernetztes Polyvinylpyrrolidon, vernetzte Polyacrylate oder Kombinationen aus Säuren, beispielsweise Zitronensäure, mit Natriumcarbonat.

Die vorliegende Erfindung betrifft auch die Verwendung der erfindungsgemäßen Polyolalkylether als Tenside. Die erfindungsgemäßen Polyolalkylether können auch als Co-Tenside in Mischung mit anderen Tensiden eingesetzt werden.

Neben der Verwendung der Polyolalkylether als Tenside in den oben genannten Wasch- und Reinigungsmitteln können diese auch mit Vorteil für eine Vielzahl anderer chemisch-technischer Prozesse eingesetzt werden

Die vorliegende Erfindung betrifft auch die Verwendung der erfindungsgemäßen Polyolalkylether als Tenside in Wasch- und Reinigungsmitteln, in der metallverarbeitenden Industrie, bei der Herstellung und Verarbeitung von Textilien, in der Leder-, Papier-, Druck-, Galvano- und Photoindustrie, bei der Wasserbehandlung, in Pflanzenschutz-Formulierungen oder in der kunststoffherstellenden und kunststoffverarbeitenden Industrie.

Die Verwendung der erfindungsgemäßen Polyolalkylether in der metallverarbeitenden Industrie umfasst beispielsweise die Verwendung in
- Kühlschmierstoffen,
- Härteölen,
- Hydraulikölemulsionen,
- Polierpasten,
- Formtrennmitteln,
- Ziehölen,
- Beizmitteln,
- Metallreinigern,
- Metalltrocknem.

Hierbei können die Tenside speziell in den Prozessen mit Vorteil eingesetzt werden, in denen es auf eine hohe thermische Stabilität ankommt.

Weiterhin können die Tenside bei der Herstellung und Verarbeitung von Textilien eingesetzt werden. Die Anwendung von Tensiden bei der Herstellung und Verarbeitung von Textilien ist außerordentlich vielseitig, sie erstreckt sich hauptsächlich auf die Gebiete
- Vorbehandlungsmittel von Fasern,
- Herstellung von Reyon-Fasern,
- Spinnpräparation und Textilschmelzen,
- Färbehilfsmittel,
- Avivagen,
- Hydrophobiermittel,
- Hilfsmittel für den Druck,
- Antistatika,
- Beflockungs- und Beschichtungsmittel.

Weiterhin können die Tenside in der Leder-, Papier-, Druck-, Galvano- und Photoindustrie verwendet werden. Wichtige Anwendungsgebiete hierbei sind Lacke, Pigmente und Druckfarben. Eingesetzt werden Tenside in diesen Anwendungsgebieten sowohl in wässrigen als auch in nichtwässrigen Systemen. In nichtwässrigen Systemen dienen sie vor allem als Dispergierhilfsmittel, Antiabsetzmittel oder Verlaufshilfsmittel. Zudem ermöglichen Tenside die Herstellung von so genannten High-Solids-Systemen, in denen sie neben der Stabilisierung der durch Emulsionspolymerisation oder -polykondensation hergestellten Bindemittel auf Kunststoffdispersionsbasis als auch als Dispergierhilfsmittel oftmals eingesetzter organischer und anorganischer Pigmente dienen. Daneben verbessern die die Hafteigenschaften dieser Anstrichmittel.

Weiterhin können die Tenside bei der Wasserbehandlung, beispielsweise bei der Abwasserreinigung, verwendet werden.

Weiterhin können die Tenside in Pflanzenschutz-Formulierungen eingesetzt werden.

Die Polyolalkylether können auch als Tenside oder Emulgatoren in der kunststoffherstellenden und kunststoffverarbeitenden Industrie verwendet werden. Hauptanwendungsgebiete bei der Kunststoffherstellung und -verarbeitung sind
- Herstellung von Kunststoffdispersionen,
- Herstellung von Perlpolymerisationen,
- Herstellung von Schaumstoffen,
- Verwendung von grenzflächenaktiven Formtrennmitteln,
- Herstellung von Mikrokapseln,
- Verbesserung der Adhäsion zwischen Füll- und Kunststoffen,
- Zusätze zu Kunststoffdispersionen zur Erzielung besonderer Effekte wie Verschäumbarkeit, Füllstoffverträglichkeit oder Netzvermögen,
- Emulgatoren für nichtwässrige Systeme,
- Anfärben von Kunststoffen,
- Antistatische Ausrüstung von Kunststoffen,
- Klebstoffe.

### Beispiele

### Beispiel 1

6,6 g (0,36 mol) 1-Dodecen und 33,15 g (0,24 mol) Glycerin werden unter Stickstoffatmosphäre in einem 250 ml-Glaskolben eingewogen. Zu dieser Reaktionsmischung werden 2,2 g Katalysator (β-Zeolith von PQ) gegeben. Mittels eines Präzisionsrührers wird die Reaktionsmischung bei 150 °C drei Stunden heftigst gerührt. Die Phasentrennung erfolgt in einem Scheidetrichter. Das Produkt wird aus der olefinischen Phase durch Abdestillation des nicht umgesetzten Olefins gewonnen, sowohl nicht umgesetztes Olefin wie auch nicht umgesetztes Glycerin können in die Reaktion zurückgeführt werden.

### Beispiel 2

64,3 g Diglycerin (99%) (0,39 mol) und 64,3 g Diglyme werden unter Stickstoffatmosphäre in einen 250 ml Glaskolben eingewogen und auf 100 °C erwärmt. Zu dieser Mischung werden im Stickstoffgegenstrom 13 g 1-Dodecen (0,08 mol) und 1,9 g Katalysator (β-Zeolith von Zeolist) gegeben. Mittels eines Präzisionsrührers wird die Mischung bei 150 °C sechs Stunden heftig gerührt. Nach dem Abkühlen der Reaktionsmischung erfolgt die Phasentrennung in einem Scheidetrichter. Durch Destillation wird das Additionsprodukt aus der olefinischen Phase gewonnen. Nicht umgesetztes Diglycerin und Olefin können in die Reaktion zurückgeführt werden.

### Beispiel 3

Polyglycerol-3 wird durch Erhitzen von Glycerin (99,5 %) mit NaOH (100 %) auf 230 °C hergestellt. Das Reaktionswasser wird dabei kontinuierlich entfernt. Die Reaktion wird bei Erreichen der gewünschten Hydroxylzahl (1169) durch schnelles Abkühlen auf 80 °C gestoppt. Das Polyglycerol-3 wird anschließend mit Wasser verdünnt und über einen Ionentauscher neutralisiert. Das Wasser entfernt man nach Zugabe von Toluol am Wasserauskreiser.

17,5 g 1-Dodecen (0,104 mol), 52,0 g Polyglycerol-3 (0,217 mol bei mittlerer molarer Masse von 240 g/mol;) und 104 g 1,4-Dioxan werden in einen Rührautoklaven eingewogen und unter Stickstoff gesetzt. Anschließend werden 1,26 g Katalysator (β-Zeolith von Zeolyst) zugegeben und die Mischung auf 150 °C erwärmt. Über einen Zeitraum von 24 h wird die Reaktionsmischung bei dieser Temperatur heftig gerührt. Nach dem Abkühlen der Reaktionsmischung werden die zwei Phasen im Scheidetrichter getrennt. Das in der oberen, organischen Phase enthaltene Additionsprodukt wird aus dieser durch destillative Entfernung des Lösungsmittels und des unumgesetzten Olefins gewonnen. Das Additionsprodukt kann bei Bedarf durch wässrige Extraktion von restlichem Polyglycerol gereinigt werden.

## Patentansprüche

1. Verfahren zur Herstellung von Polyolalkylethern durch Umsetzung von wenigstens drei Hydroxyfunktionalitäten enthaltenden Verbindungen mit Olefinen in Gegenwart von sauren Katalysatoren bei Temperaturen von 20 bis 250 °C und Drücken von 0,5 bis 10 bar, **dadurch gekennzeichnet, dass** die Olefine der allgemeinen Formel (I) entsprechen 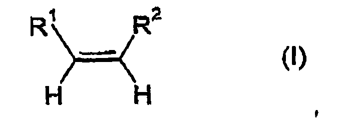 worin R¹ Wasserstoff und R² linearer oder verzweigter Kohlenstoffrest mit 7 bis 28 Kohlenstoffatomen,
oder
R¹ und R² jeweils linearer oder verzweigter Kohlenstoffrest mit 1 bis 27 Kohlenstoffatomen, wobei die Summe der Kohlenstoffanzahl von R¹ und R² höchstens 28 beträgt, bedeuten, wobei die wenigstens drei Hydroxyfunktionalitäten enthaltende Verbindung ausgewählt ist aus Sorbitol, Trimethylolpropan, Pentaerythrit, Verbindungen der Formel (IV), (V). (VI), (IX) und Mischungen davon 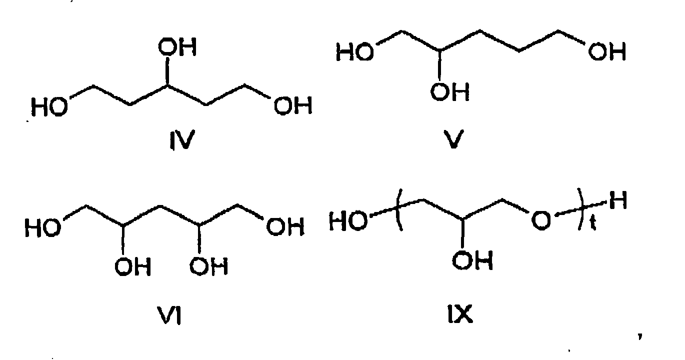 wobei t 1 bis 20 ist und die Verbindungen der Formel (IX) linear oder verzweigt sein können.

2. Verfahren nach Anspruch 1, **dadurch gekennzeichnet, dass** es in Anwesenheit eines Lösungsmittels durchgeführt wird.

3. Verfahren nach Anspruch 1 oder 2, **dadurch gekennzeichnet, dass** Olefine ausgewählt aus der Gruppe bestehend aus internen, verzweigten Olefinen mit der Kettenlänge C₁₂, linearen oder verzweigten α-Olefinen der Kettenlänge C₁₂ und C₁₄ und Mischungen davon eingesetzt werden.

4. Verfahren nach Anspruch 3, **dadurch gekennzeichnet, dass** ein C₁₂-, ein C₁₄-α-Olefin oder eine Mischung eines C₁₂- und eines C₁₄-α-olefins eingesetzt wird.

5. Verfahren nach einem der Ansprüche 1 bis 4, **dadurch gekennzeichnet, dass** der saure Katalysator ein Zeolith ist.

6. Polyolalkylether, abgeleitet von Verbindungen mit wenigstens drei Hydroxyfunktionalitäten ausgewählt aus Verbindungen der Formel (IV), (V), (VI), (IX) und Mischungen davon 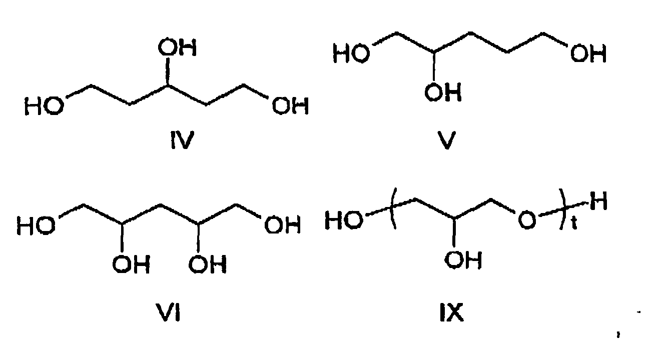 wobei t 2 bis 20 ist, und die Verbindungen der Formel (IX) linear oder verzweigt sein können, wobei höchstens alle bis auf eine Hydroxyfunktionalität durch eine Gruppierung der allgemeinen Formel (VIII) 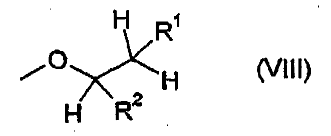 ersetzt sind, worin R¹ Wasserstoff und R² linearer oder verzweigter Kohlenstoffrest mit 7 bis 28 Kohlenstoffatomen,
oder
R¹ und R² jeweils linearer oder verzweigter Kohlenstoffrest mit 1 bis 27 Kohlenstoffatomen, wobei die Summe der Kohlenstoffanzahl von R¹ und R² höchstens 28 beträgt, bedeuten.

7. Verwendung von Polyolalkylethern abgeleitet von Verbindungen mit wenigstens drei Hydroxyfunktionalitäten, wobei höchstens alle bis auf eine Hydroxyfunktionalität durch eine Gruppierung der allgemeinen Formel (VIII) 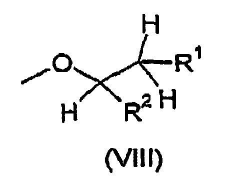 ersetzt wird, worin R¹ Wasserstoff und R² linearer oder verzweigter Kohlenstoffrest mit 7 bis 28 Kohlenstoffatomen
oder
R¹ und R² jeweils linearer oder verzweigter Kohlenstoffrest mit 1 bis 27 Kohlenstoffatomen, wobei die Summe der Kohlenstoffanzahl von R¹ und R² höchstens 28 beträgt, bedeuten und die wenigstens drei Hydroxyfunktionalitäten enthaltende Verbindung ausgewählt ist aus Sorbitol, Trimethylolpropan, Pentaerythrit, Verbindungen der Formel (IV), (V), (VI), (IX) und Mischungen davon 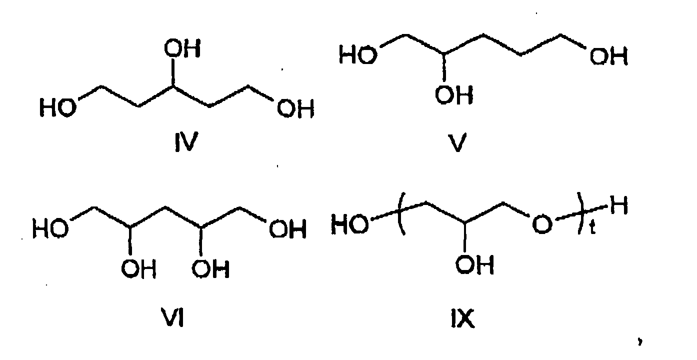 wobei t 2 bis 20 ist und die Verbindungen der Formel (IX) linear oder verzweigt sein können als Tenside in Wasch- und Reinigungsmitteln, in der metailverarbeitenden Industrie, bei der Herstellung und Verarbeitung von Textilien, in
der Leder-, Papier-, Druck-, Galvano- und Photoindustrie, bei der Wasserbehandlung, in Pflanzenschutz-Formulierungen oder in der kunststoffherstellenden und kunststoffverarbeitenden industrie.

8. Wasch- und Reinigungsmittel, **dadurch gekennzeichnet, dass** sie Polyolalkylether nach Anspruch 6 enthalten.

## Claims

1. A process for preparing polyol alkyl ethers by reacting compounds comprising at least three hydroxyl functionalities with olefins in the presence of acidic catalysts at temperatures of from 20 to 250°C and pressures of from 0.5 to 10 bar, wherein the olefins correspond to the general formula (I) 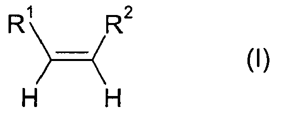 in which R¹ is hydrogen and R² is a linear or branched carbon radical having from 7 to 28 carbon atoms,
or
R¹ and R² are each linear or branched carbon radicals having from 1 to 27 carbon atoms, the sum of the carbon number of R¹ and R² being at most 28, the compound comprising at least three hydroxyl functionalities being selected from sorbitol, trimethylolpropane, pentaerythritol, compounds of the formula (IV), (V), (VI), (IX) and mixtures thereof. 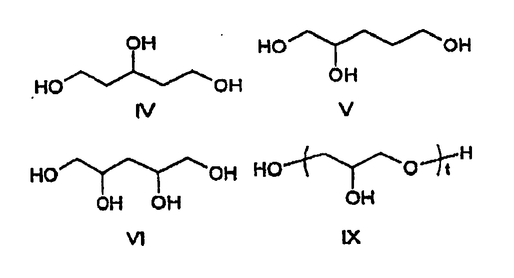 where t is from 1 to 20 and the compounds of the formula (IX) may be linear or branched.

2. The process according to claim 1, which is carried out in the presence of a solvent.

3. The process according to claim 1 or 2, wherein olefins selected from the group consisting of internal, branched olefins of chain length C₁₂, linear or branched α-olefins of chain length C₁₂ and C₁₄, and mixtures thereof are used.

4. The process according to claim 3, wherein a C₁₂-α-olefin, a C₁₄-α-olefin or a mixture of a C₁₂- and of a C₁₄-α-olefin is used.

5. The process according to any of claims 1 to 4, wherein the acidic catalyst is a zeolite.

6. A polyol alkyl ether derived from compounds having at least three hydroxyl functionalities selected from compounds of the formula (IV), (V), (VI), (IX) and mixtures thereof 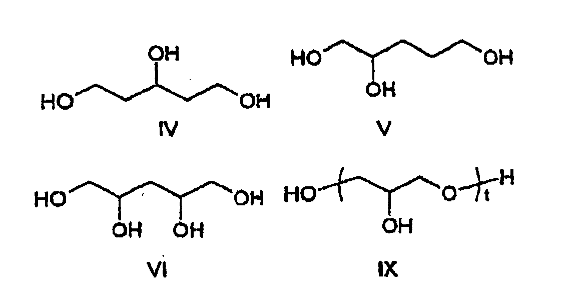 where t is from 2 to 20, and the compounds of the formula (IX) may be linear or branched, not more than all but one hydroxyl functionality being replaced by a moiety of the general formula (VIII) 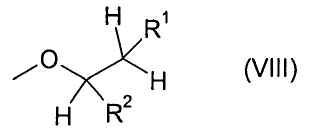 in which R¹ is hydrogen and R² is a linear or branched carbon radical having from 7 to 28 carbon atoms,
or
R¹ and R² are each linear or branched carbon radicals having from 1 to 27 carbon atoms, the sum of the carbon number of R¹ and R² being at most 28.

7. The use of polyol alkyl ethers derived from compounds having at least three hydroxyl functionalities, not more than all but one hydroxyl functionality being replaced by a moiety of the general formula (VIII) 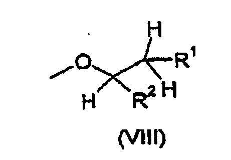 in which R¹ is hydrogen and R² is a linear or branched carbon radical having from 7 to 28 carbon atoms,
or
R¹ and R² are each linear or branched carbon radicals having from 1 to 27 carbon atoms, the sum of the carbon number of R¹ and R² being at most 28, and the compound comprising at least three hydroxyl functionalities being selected from sorbitol, trimethylolpropane, pentaerythritol, compounds of the formula (IV), (V), (VI), (IX) and mixtures thereof 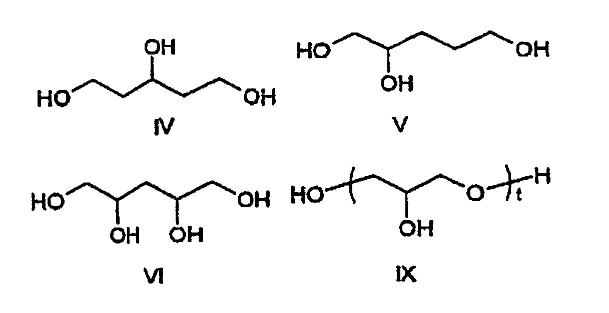 where t is from 2 to 20 and the compounds of the formula (IX) may be linear or branched, as surfactants in laundry detergents and cleaning compositions, in the metal processing industry, in the production and processing of textiles, in the leather industry, paper industry, printing industry, electroplating industry and photographic industry, in water treatment, in crop protection formulations or in the plastics production industry and plastics processing industry.

8. A laundry detergent or cleaning composition, which comprises polyol alkyl ethers according to claim 6.

## Revendications

1. Procédé pour la préparation de polyalkyléthers par mise en réaction de composés contenant au moins trois fonctionnalités hydroxy avec des oléfines en présence de catalyseurs acides, à des températures de 20 à 250 °C et sous des pressions de 0,5 à 10 bars, **caractérisé en ce que** les oléfines correspondent à la formule générale (I) 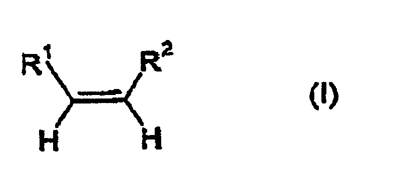 dans laquelle R¹ représente un atome d'hydrogène et R² représente un radical carboné linéaire ou ramifié ayant de 7 à 28 atomes de carbone,
ou
R¹ et R² représentent chacun un radical carboné linéaire ou ramifié ayant de 1 à 27 atomes de carbone, la somme des nombres d'atomes de carbone de R¹ et R² étant de 28 au maximum, le composé contenant au moins trois fonctionnalités hydroxy étant choisi parmi le sorbitol, le triméthylolpropane, le pentaérythritol, des composés de formules (IV), (V), (VI), (IX) et des mélanges de ceux-ci 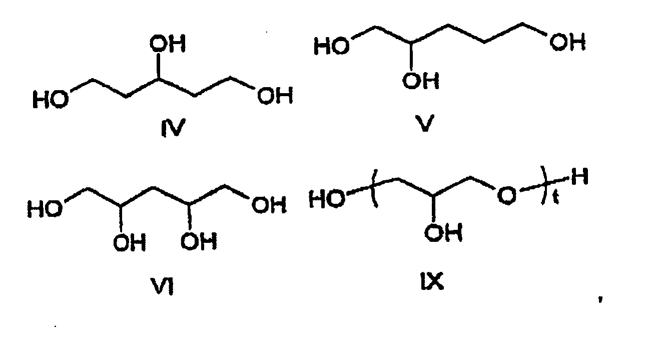 t allant de 1 à 20 et les composés de formule (IX) pouvant être linéaires ou ramifiés.

2. Procédé selon la revendication 1, **caractérisé en ce qu'**il est effectué en présence d'un solvant.

3. Procédé selon la revendication 1 ou 2, **caractérisé en ce que** les oléfines sont choisies dans le groupe constitué par des oléfines ramifiées internes ayant une longueur de chaîne en C₁₂, des α-oléfines linéaires ou ramifiées ayant une longueur de chaîne en C₁₂ ou C₁₄ et des mélanges de celles-ci.

4. Procédé selon la revendication 3, **caractérisé en ce qu'**on utilise une α-oléfine en C₁₂, une α-oléfine en C₁₄ ou un mélange d'une oléfine en C₁₂ et d'une α-oléfine en C₁₄.

5. Procédé selon l'une quelconque des revendications 1 à 4, **caractérisé en ce que** le catalyseur acide est une zéolithe.

6. Polyalkyléther, dérivé de composés comportant au moins trois fonctionnalités hydroxy, choisi parmi les composés de formules (IV), (V), (VI) (IX) et des mélanges de ceux-ci 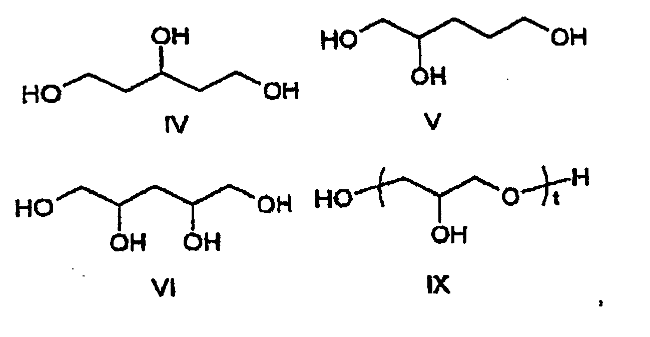 t allant de 2 à 20 et les composés de formule (IX) pouvant être linéaires ou ramifiés, au maximum toutes les fonctionnalités hydroxy sauf une étant remplacées par un groupement de formule générale (VIII) 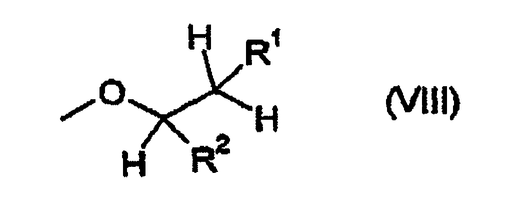 dans laquelle R¹ représente un atome d'hydrogène et R² représente un radical carboné linéaire ou ramifié ayant de 7 à 28 atomes de carbone,
ou
R¹ et R² représentent chacun un radical carboné linéaire ou ramifié ayant de 1 à 27 atomes de carbone, la somme des nombres d'atomes de carbone de R¹ et R² étant de 28 au maximum.

7. Utilisation de polyalkyléthers dérivés de composés comportant au moins trois fonctionnalités hydroxy, au maximum toutes les fonctionnalités hydroxy sauf une étant remplacées par un groupement de formule générale (VIII) 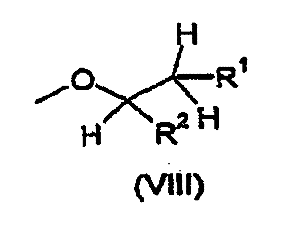 dans laquelle R¹ représente un atome d'hydrogène et R² représente un radical carboné linéaire ou ramifié ayant de 7 à 28 atomes de carbone,
ou
R¹ et R² représentent chacun un radical carboné linéaire ou ramifié ayant de 1 à 27 atomes de carbone, la somme des nombres d'atomes de carbone de R¹ et R² étant de 28 au maximum, et le composé contenant au moins trois fonctionnalités hydroxy étant choisi parmi le sorbitol, le triméthylolpropane, le pentaérythritol, des composés de formules (IV), (V), (VI), (IX) et des mélanges de ceux-ci 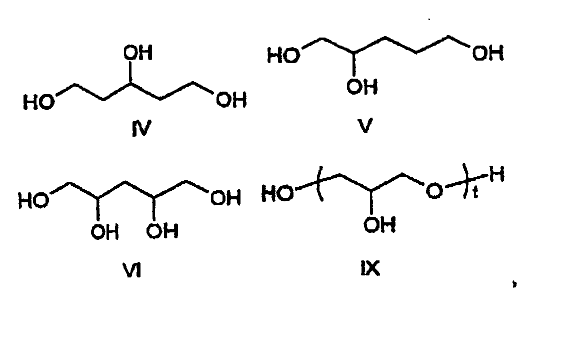 t allant de 2 à 20 et les composés de formule (IX) pouvant être linéaires ou ramifiés, en tant que tensioactifs dans des produits de lavage et de nettoyage, dans l'industrie de transformation des métaux, dans la fabrication et le traitement de textiles, dans l'industrie du cuir, du papier, de l'imprimerie, galvanoplastique et photographique, dans le traitement de l'eau, dans des compositions phytosanitaires ou dans l'industrie de fabrication des matières plastiques et de transformation des matières plastiques.

8. Produits de lavage et de nettoyage, **caractérisés en ce qu'**ils contiennent des polyalkyléthers selon la revendication 6.
